# EUROPEAN PATENT APPLICATION

(11) **EP 4 047 101 A1**
(43) Date of publication of application: **24.08.2022**
(21) Application number: 19949066.5
(22) Date of filing: 22.10.2019
(51) Int. Cl.: C12Q 1/6886, C12Q 1/6858, C12N 15/12

(54) **PRIMER SET FOR METHYLATION OF A SPECIFIC REGION OF A HUMAN COLORECTAL CANCER-RELATED GENE, TEST, TEST KIT AND APPLICATION THEREOF**

(30) Priority: 16.10.2019 CN 201910982813
(71) Applicant: Xiamen Sciendox Biological Technology Co., Ltd., Haicang District, Xiamen, Fujian 361006 (CN)
(72) Inventor: YAN, Hongli, Xiamen, Fujian 361006 (CN); YANG, Luping, Xiamen, Fujian 361006 (CN); SONG, Lihua, Xiamen, Fujian 361006 (CN); LIAO, Qinghua, Xiamen, Fujian 361006 (CN)
(74) Representative: Kuhnen & Wacker Patent- und Rechtsanwaltsbüro PartG mbB
(86) International application number: PCT/CN2019/112439
(87) International publication number: WO 2021/072786

(57) **Abstract**

The present application discloses a primer set, a reagent, a kit for detecting methylation of a specific region of a human colorectal cancer-related gene from feces, blood, or tissue by using specific PCR, and the application thereof. The primer set includes specific amplified primer probe sequences for the methylated regions of specific regions of the gene markers PRDM12, FOXE1, and B3GAT2 genes found in this study; and the primer probe group also includes the specific amplification primer probe sequence of the methylated region of the specific region of the Vimentin, SFRP2-1, and SFRP2-2 genes and the specific amplification primer probe group of the internal reference gene ACTB, thus having high detection efficiency and strong pertinence.

## Description

### TECHNICAL FIELD

The present application relates to the field of biotechnology, and more particularly to a primer set, a reagent, a kit for detecting methylation of specific regions of human colorectal cancer-related genes from feces, blood or tissue by using specific PCR, and application of the kit, which are suitable for detecting human genome to determine whether methylation occurs in specific regions of colorectal cancer and precancerous adenoma-related genes.

### BACKGROUND

Colorectal cancer, also known as advanced adenoma, is a common malignant tumor of the digestive system. The current research results believe that the occurrence of colorectal cancer is not only related to genetic factors, presenting obvious family inheritance tendency, but also related to the influence of various environmental factors. Colorectal mucosal epithelial cells firstly have genetic and epigenetic changes under the combined action of a variety of factors to acquire the ability of malignant proliferation, invasion, infiltration, and metastasis, then form local masses, and finally form metastases in lymph nodes and other tissues and organs. In recent years, with the improvement of people's living standards, changes in dietary structure and habits, prolongation of life expectancy, and the trend of population aging, the incidence and mortality of colorectal cancer in my country have shown a linear upward trend, and the age of onset of disease has an advanced trend. According to the "2015 China Cancer Statistics" published in "CA: A Cancer Journal for Clinicians", colorectal cancer ranks fifth in male cancer incidence and fourth in female cancer incidence; and colorectal cancer ranks fifth in cancer mortality in both men and women. Although the current surgical methods and adjuvant treatment methods have been continuously improved, the postoperative survival of colorectal cancer patients has been greatly improved, but the five-year overall survival rate of patients is still not ideal, which is mainly because that the survival rate and tumor stage of colorectal cancer patients are closely related, the 5-year survival rate of patients with early-stage colorectal cancer after surgery exceeds 90%, but most of the patients have developed to the middle or advanced stage of the tumor when they have symptoms or discover the disease. Since 93% of colorectal cancers originate from adenomas, most of them have no characteristic symptoms in the early stage, and it takes 3 to 17 years to develop from adenomas to cancer, which provides a time basis for early screening. Therefore, screening of high-risk population of colorectal cancer, early detection and diagnosis of colorectal cancer patients, and early treatment of colorectal cancer patients are important aspects to improve the overall survival rate of patients.

The existing screening methods for colorectal cancer and advanced adenoma are mainly divided into two categories: feces test and colon structural test. Feces testing includes: fecal occult blood test (FOBT) and stool DNA testing (sDNA). Colon structural testing includes: flexible sigmoidoscopy (FSIG), colonoscopy (CS), double contrast barium enema (DCBE), and CT colonoscopy (CTC). FOBT is a simple and rapid screening method for colorectal cancer and advanced adenomas, and is used to detect trace hemoglobin in feces samples. FOBT is simple, inexpensive, and non-invasive, and can be used for large-scale screening studies. However, FOBT can be influenced by many factors. Positive results can only be detected when the tumor ruptures and bleeds, while the bleeding of polyps or cancer tissue is often intermittent and cannot be contained in any feces sample. Therefore, FOBT has poor specificity and may result in missed diagnosis. FSIG is a method of directly examining the distal colon using endoscopic techniques. The operation is relatively simple, the bowel preparation requirements are relatively low, and intraoperative sedation is not required, and lesions can be biopsied and treated, which make FSIG a relatively inexpensive, simple, and safe screening method. However, the inspection site of FSIG is limited to the distal large bowel (bounded by the splenic flexure), and if colonoscopy is performed after FSIG found distal colorectal tumors, 72.0% of proximal colorectal tumors would be missed. Colonoscopy (CS) is the current gold standard for the diagnosis and treatment of colorectal cancer and advanced adenomas, and allows a complete observation of the entire colon, biopsy and removal of polyps found. Although CS has the advantages of good curative effect and high accuracy, it also has certain limitations: (1) CS is an invasive examination method with a higher risk of complications, which may cause colon perforation (0.3%) or even death (1/5000); (2) time-consuming, requiring adequate bowel preparation and sedation before examination; (3) expensive, if biopsy is required and polyps is to be removed, the cost will be higher; (4) colonoscopy has a missed diagnosis rate of 5% for colorectal cancer and advanced adenoma. CTC is a non-invasive procedure that scans tomography at a thickness of 1-2 mm to detect intestinal lesions on 2D and 3D images. CTC has the advantages of rapidity, accuracy, and low complication, and its screening efficiency for larger polyps, colorectal cancer, and advanced adenoma is close to that of CS. However, as a routine screening method, CTC has obvious shortcomings: in order to obtain a satisfactory image, CTC still needs to release 2-4 rads X-rays to the patient's abdomen during the examination, which increases the risk of cancer; usually the injection of air or carbon dioxide into the intestinal lumen to dilate the intestinal tract will cause some discomfort to the patient during examination; in addition, the high examination cost will bring a heavier economic burden to both individuals and society.

Stool exfoliated cells and genetic testing (sDNA) is currently the most cutting-edge technology for colorectal cancer and advanced adenoma screening. It is a non-invasive screening method. Compared with normal colorectal mucosa, the metabolic rate of colorectal cancer and advanced adenoma cells was significantly increased, and the exfoliated cells increased accordingly. Through quantitative analysis of the DNA of exfoliated cells in the feces, some colorectal cancers and advanced adenomas can be effectively screened. This method generally includes two types of tests: the first type is the detection of fecal exfoliation cytology, that is, routine pathological examination of the exfoliated intestinal epithelial cells in the feces to find out the tumor cells, so as to achieve a clear diagnosis of intestinal tumors. diagnosis. The second type is the detection of fecal exfoliated cell DNA. Because studies have shown that the mutated genes in the exfoliated tumor cells in the patient's feces are highly consistent with the mutated genes in the tumor tissue itself. If this type of gene can be detected from the tumor cells exfoliated from the feces, the diagnosis of intestinal tumors can also be confirmed. The method has high specificity, and a positive result can basically confirm the existence of lesions. There is no need to change the dietary habits of the screening population, or to restrict the use of drugs.

The PRDM12 gene (PRDI-BF1 and RIZ homology domain-containing protein 12) is located in human chromosome 9 9q34.12 (chr9: 130,664,594 - 130,682,997, hg38) and belongs to a family of transcriptional regulators containing PR and SET protein domains (PR/SET domain family). The PRDM12-encoded protein contains N-terminal PRDI-BF1 and RIZ homology domains (PR), one SET domain, and three C2H2 zinc finger DNA binding domains at the C-terminal. Domestic and foreign studies have shown that PRDM12 is an important transcriptional regulator that controls neural differentiation and formation, and is associated with the occurrence of solid tumors and hematological malignancies. At present, there is no research report on the relationship between the methylation degree of specific regions of the PRDM12 gene and tumorigenesis at home and abroad, and there is no research report on the quantitative detection kit for the quantitative detection of the methylation degree of PRDM12.

FOXE1 gene (Forkhead Box E1) is located in human chromosome 9 9q22.33 (chr9:97,853,254-97,856,715, hg38), is a member of the forkhead transcription factor family (Forkhead family), and contains the common forkhead domain of the family genes (Forkhead domain). This domain consists of more than 100 amino acids, which can recognize and bind to specific sequences of genomic DNA. Previous studies have shown that FOXE1 is an important transcription factor, and a thyroid-specific forkhead transcription factor, which is essential for thyroid morphogenesis and differentiation, and is also involved in thyroid hormone-induced transcriptional regulation. FOXE1 is directly related to the occurrence of thyroid cancer, pancreatic cancer, and skin squamous cell carcinoma. Hypermethylation of FOXE1 gene has also been reported in skin squamous cell carcinoma and breast cancer. Although the role of FOXE1 in colorectal cancer is still unclear, the latest research has found that abnormally high methylation of FOXE1 gene causes low gene expression, which is related to colorectal cancer metastasis and patient prognosis, and has been used as a potential biomarker for colorectal cancer detection ( biomarker). Although there are reports on the detection of methylation in specific regions of the FOXE1 gene in the above studies, there is no report on the quantitative detection kits for the quantitative detection of methylation in specific regions of the FOXE1 gene at home and abroad.

The B3GAT2 gene (Beta-1,3-Glucuronyltransferase 2) is located in human chromosome 6 6q13 (chr6:70,856,679-70,957,189, hg38), a member of the glucuronyltransferase family, and encodes a transmembrane protein. The main function of B3GAT2 gene is catalyzing Beta-1,3-linked glucuronic acid to be transferred to the terminal galactosyl group of Gal-Beta-1-4 GLcNAc or Gal-Beta-1-3 GLcNAc residues contained in various glycoproteins or glycolipids. Previous studies have found that B3GAT2 is involved in the synthesis of HNK-1 sugar chain epitopes, which is related to cell recognition, adhesion, and migration in the nervous system, and has a regulatory effect on the growth and development of the nervous system, neuron regeneration, and synaptic plasticity. At present, there is no research report on the relationship between B3GAT2 gene and tumorigenesis at home and abroad. Only one study finds that the methylation of a specific region of B3GAT2 gene is related to the occurrence of Barrett's Esophagus, which has been used as a biomarker for the diagnosis of this disease. Although Barrett's esophagus has the potential to develop into esophageal cancer, the risk of canceration is very low. At present, there are no reports at home and abroad on a detection kit for quantitatively detecting the methylation degree of specific regions of the B3GAT2 gene.

The Vimentin gene is located on chromosome 10, 10P13 (chr10:17,229,548-17,229,607), which belongs to the type III intermediate filament fibrin gene. The Vimentin gene is a member of the intermediate filament fibrin family and exhibits complex gene expression patterns in many respects, and are mainly expressed in embryonic tissues and adult cells derived from mesenchymal tissue. More and more studies have shown that Vimentin expression is not a specific biomarker for mesenchymal origin cells and mesenchymal differentiation tumor cells. It has been found that keratin and Vimentin are co-expressed in breast cancer, hepatocellular carcinoma, prostate cancer, endometrial cancer, gastric cancer, esophageal cancer, colon cancer, lung cancer, and other epithelial-derived malignant tumors, and are closely related to strong invasiveness and easy transferring ability of cancer cells. Another study confirmed that the down-regulation of Vimentin, which is associated with the production of tumors and precancerous lesions (such as adenomas), may be closely related to the methylation of its promoter. This suggests that DNA methylation can down-regulate the expression of Vimentin from the gene transcription level, which has a promoting effect on tumor cell migration. Chen et al. showed that the methylation rates of Vimentin in early colorectal cancer and advanced adenoma tissues without metastases and in late colorectal cancer and advanced adenoma are equivalent. Other studies have shown that methylation of specific regions of the Vimentin gene is not associated with tumor size and Duke's stage. These all illustrate the importance of methylation detection in specific regions of the Vimentin gene for the early diagnosis of colorectal cancer and advanced adenoma.

The SFRP2 gene is located on chromosome 4q31.3 (chr4:153,789,388-153,789,447), and the encoded SFRP2 protein contains a characteristic cysteine-rich region. Many studies have shown that hypermethylation of the SFRP2 gene can cause the gene to be silenced in a variety of tumors. The SFRP2 gene contains two introns and three exons. There is a high density of CpG islands around the first exon of the SFRP2 gene. This CpG island is closely related to DNA hypermethylation in tumor cells. In recent years, many studies have found that SFRP2 gene hypermethylation is involved in the occurrence of colorectal cancer. Numerous studies have demonstrated that the degree of SFRP2 methylation is the most sensitive marker for the diagnosis of colorectal cancer. Therefore, detection of methylation in specific regions of the SFRP2 gene from fecal exfoliated cells can be used as one of the basis for screening colorectal cancer.

### SUMMARY

In view of the above-described problem in the existing technology, it is a first objective of the present application to provide a primer set for methylation of specific regions of human colorectal cancer-related genes. The primer set comprises: a primer set for methylation areas of any one gene selected from the group consisting of PRDM12, FOXE1, B3GAT2, Vimentin, SFRP2-1, and SFRP2-2, or any gene combination thereof.

Optionally, a detection primer set for the PRDM12 gene has a specific primer-probe set having the following sequences:
a forward primer: 5'-GTTAGATTAGAAGTATAAAATGTG-3', referred to as a primer 1;
a reverse primer: 5'-ATCCCATTCCTCTCCTCC-3', referred to as a primer 2; and
a detection probe: 5'-AGCGCGGTGGAGATTTG-3', referred to as a probe 1.

Optionally, a detection primer set for the FOXE1 gene has a specific primer-probe set having the following sequences:
a forward primer: 5'- CGAGTttAAGtttTTGGtAGAGG -3', referred to as a primer 3;
a reverse primer: 5'- CCGATCGACTaAaaCCCGa -3', referred to as a primer 4; and
a detection probe: 5'- tCGAGTTCGGGCGtTGAGG -3', referred to as a probe 2.

Optionally, a detection primer set for the B3GAT2 gene has a specific primer-probe set having the following sequences:
a forward primer: 5'- TtCGtCGGGTtTGGCG -3', referred to as a primer 5;
a reverse primer: 5'- CAaaAaaTCGTaCAaCCCCG -3', referred to as a primer 6; and
a detection probe: 5'- tCGCGAGtAAGtTCGGGAG -3', referred to as a probe 3.

Optionally, a detection primer set for the Vimentin gene has a specific primer-probe set having the following sequences:
a forward primer: 5'- GtCGtAGttTtTACGttTCGT -3', referred to as a primer 7;
a reverse primer: 5'- aaCGCACGaCAaAaaAaCG -3', referred to as a primer 8; and
a detection probe: 5'- ttCGGGCGGCGTGTATG -3', referred to as a probe 4.

Optionally, a detection primer set for the SFRP2-1 gene has a specific primer-probe set having the following sequences:
a forward primer: 5'- CGGAtTGGGGtAAAAtAAGttC -3', referred to as a primer 9;
a reverse primer: 5'- CTaaCGaaAACGCGCCTA -3', referred to as a primer 10; and
a detection probe: 5'- TAGGCGCGTTttCGttAGTAttTGG -3', referred to as a probe 5.

Optionally, a detection primer set for the SFRP2-2 gene has a specific primer-probe set having the following sequences:
a forward primer: 5'- AATGtAGtCGGCGCG -3', referred to as a primer 11;
a reverse primer: 5'- CCTTaTTaaaAaTTCAAaaAaCCCG -3', referred to as a primer 12; and
a detection probe: 5'- AGttAtTTtCGGGCGTGCGGt -3', referred to as a probe 6.

Optionally, the primer set further comprises a primer set for an internal reference ACTB gene. the primer set for the internal reference ACTB gene has a specific primer-probe set having the following sequences:
a forward primer: 5'- GTGATGGAGGAGGTTTAGTAAG -3', referred to as a primer 13;
a reverse primer: 5'- CAATAAAACCTACTCCTCCCTT -3', referred to as a primer 14; and
a detection probe: 5'- TGTGTTTGTTATTGTGTGTTGGGTGGT -3', referred to as a probe 7.

Optionally, a fluorescent reporter group at a 5' end of the detection probe comprises: ALEX-350, FAM, VIC, TET, CAL Fluor Gold 540, JOE, HEX, CAL Flour Orange 560, TAMRA, Cal Fluor Red 590, ROX, CAL Fluor 20 Red 610, TEXAS RED, CAL Flour Red 635, Quasar 670, Cy3, Cy5, Cy5.5, or Quasar 705. A quenching group at 3' end of the detection probe comprises: TAMRA, BHQ1, BHQ2, and MGB.

It is another objective of the present application to provide a reagent for detecting methylation of specific regions of human colorectal cancer-related genes. The reagent comprises: six PCR systems, that are a CRC PCR Mix-A, a CRC PCR Mix-B, a CRC PCR Mix-C, a CRC PCR Mix-D, a CRC PCR Mix-E, and a CRC PCR Mix-F, respectively. Each PCR system comprises: the specific primer-probe set for the methylation of specific regions of a corresponding gene as described in the above; and the specific primer-probe set for the internal reference gene as described in the above. The PCR system A comprises: a forward primer (primer 1) for the PRDM12 gene, a reverse primer (primer 2) for the PRDM12 gene, and a detection probe (probe 1) for the PRDM12 gene. The PCR system B comprises: a forward primer (primer 3) for the FOXE1 gene, a reverse primer (primer 4) for the FOXE1 gene, and a detection probe (probe 2) for the FOXE1 gene. The PCR system C comprises: a forward primer (primer 5) for the B3GAT2 gene, a reverse primer (primer 6) for the B3GAT2 gene, and a detection probe (probe 3) for the B3GAT2 gene. The PCR system D comprises: a forward primer (primer 7) for the Vimentin gene, a reverse primer (primer 8) for the Vimentin gene, and a detection probe (probe 4) for the Vimentin gene. The PCR system E comprises: a forward primer (primer 9) for the SFRP2-1 gene, a reverse primer (primer 10) for the SFRP2-1 gene, and a detection probe (probe 5) for the SFRP2-1 gene. The PCR system F comprises: a forward primer (primer 11) for the SFRP2-2 gene, a reverse primer (primer 12) for the SFRP2-2 gene, and a detection probe (probe 6) for the SFRP2-2 gene.

Optionally, each of the six PCR systems, that are a CRC PCR Mix-A, a CRC PCR Mix-B, a CRC PCR Mix-C, a CRC PCR Mix-D, a CRC PCR Mix-E, and a CRC PCR Mix-F, further comprises general ingredients, including a PCR buffer, dNTPs, and MgCl₂.

Optionally, the internal reference gene is a human housekeeping gene, that is, the ACTB gene. The primers and the probe of the internal reference gene are used to monitor the quality of each sample and its amplification in each reaction system, thereby avoiding false negative result or partially suppressed result.

Optionally, in the six PCR systems of the CRC PCR Mix-A, the CRC PCR Mix-B, the CRC PCR Mix-C, the CRC PCR Mix-D, the CRC PCR Mix-E, and the CRC PCR Mix-F, each reaction system has a final volume of between 20 and 40 µL; primer concentrations of each reaction system are as follows: the forward primer has a concentration of between 0.3 and 0.8 µM, the reverse primer has a concentration of between 0.3 and 0.8 µM, and the probe has a concentration of between 0.1 and 0.3 µM; and final concentrations of the general ingredients of each of the six PCR systems are as follows: final concentrations of dATP, dTTP, dCTP, and dGTP in the dNTPs are all between 0.1 and 0.3 mM, and a final concentration of MgCl₂ is between 1 and 2 mM.

It is still another objective of the present application to provide a kit for detecting methylation of specific regions of human colorectal cancer-related genes. The kit comprises: any one of the six PCR systems or any combination thereof; between 0.5 and 2 U of a hot-start taq polymerase; a positive control; a negative control; and a blank control. The positive control is a mixture of the internal reference ACTB gene and methylated plasmid DNA fragments of PRDM12, FOXE1, B3GAT2, Vimentin, SFRP2-1, and SFRP2-2, or is a positive control of methylated human whole genome DNA. The negative control is a mixture of the internal reference ACTB gene and unmethylated plasmid DNA fragments of PRDM12, FOXE1, B3GAT2, Vimentin, SFRP2-1, and SFRP2-2, or is a negative control of unmethylated human whole genome DNA. The blank control is a Tris-EDTA buffer or nuclease-free water having a pH = 8.5 ± 0.1.

It is still another objective of the present application to provide a method of using the kit for detecting methylation of specific regions of human colorectal cancer-related genes. DNA is directly extracted from human feces, blood, or tissues, and the DNA is converted by bisulfite treatment, a resulting converted DNA is used as a template, amplified by PCR, and accumulation of signals on the detection probe is used to achieve real-time detection of methylation of specific regions of human colorectal cancer related genes of PRDM12, FOXE1, B3GAT2, Vimentin, SFRP2-1, and SFRP2-2 genes. At the same time, the ACTB gene was used as the internal reference gene. The method comprises the following specific PCR experimental steps:
step 1: collecting feces, blood, or tissue samples from a certain number of colorectal cancer patients and advanced adenoma patients;
step 2: extracting human genomic DNA from the feces, blood or tissue samples of patients to be tested;
step 3: converting a resulting extracted human genomic DNA by bisulfite, and using a resulting converted DNA as the template for the PCR;
step 4: collecting 29.7 µL ×(n+1) PCR Mix and 0.3µL ×(n+1) hot-start taq polymerase mixed solution from the detection kit, mixing uniformly for 20s, and centrifuging instantaneously for 10s; in which, n=number of samples to be tested;
step 5: dispensing a resulting centrifuged solution to PCR tubes with each PCR tube containing 30 µL of the resulting centrifuged solution, then adding 10 µL of the converted DNA template to be detected, wherein a total volume is 40 µL;
step 6: performing an amplification reaction, wherein the reaction procedure is as follows:

| stages | cycle number | reaction temperature | reaction time |
|---|---|---|---|
| first stage | 1 | 95.0°C | 1 min |
| second stage | 10 | 95.0°C | 20 s |
| | | 65.0°C | 30 s (-0.5°C/Cycler) |
| | | 72.0°C | 20 s |
| third stage | 35 | 95.0°C | 20 s |
| | | 60.0°C | 26 s (FAM, HEX, ROX, and Cy5 are lightened, and fluorescence value is detected once before the end of each cycle) |
| | | 72.0°C | 30 s |

step 7: determining PCR results, in which, during the sample detection, the baseline is determined by taking fluorescence signals of between 3 and 15 cycles; a threshold is set by making a threshold line exceeding a highest point of an amplification curve of a normal negative control, and the Ct value is Undet; positive or negative result of the sample is judge according to reported Ct values, that is, according to a cycle number experienced when the fluorescent signal in each reaction tube reaches the set threshold.

By adopting the above technical solutions, advantages of the present application are summarized as follows:
1. The present application identifies methylation of specific regions of new genes as a tumor marker for human colorectal cancer, which can be used for early screening and auxiliary detection of patients.
2. The kit of the present application selects suitable regions of the target genes and designs a highly specific primer and probe.
3. The kit of the present application can directly use feces to extract DNA, such sample collection is non-invasive, and greatly reduces the pain of the patient.
4. The present application discloses for the first time a kit for detecting the methylation of the PRDM12, FOXE1, B3GAT2, Vimentin, SFRP2-1, and SFRP2-2 genes related to colorectal cancer and advanced adenoma and application of the kit. The detection kit comprises: forward primers, reverse primers, and fluorescent probes thereof for methylation-specific amplification of the PRDM12, FOXE1, B3GAT2, Vimentin, SFRP2-1, and SFRP2-2 genes. The methylation levels of the PRDM12, FOXE1, B3GAT2, Vimentin, SFRP2-1, and SFRP2-2 genes results in low expression of the PRDM12, FOXE1, B3GAT2, Vimentin, SFRP2-1, and SFRP2-2 genes in colorectal cancer patients and advanced adenoma patients, such that the colorectal cancer cells and the advanced adenoma cells have stronger invasive ability. Therefore, the methylation status of the PRDM12, FOXE1, B3GAT2, Vimentin, SFRP2-1, and SFRP2-2 genes can be used as molecular biomarkers for the evaluation of the malignancy and prognosis of colorectal cancer and advanced adenoma. Diagnostic kits based on the detection of methylation levels of the PRDM12, FOXE1, B3GAT2, Vimentin, SFRP2-1, and SFRP2-2 genes related to the colorectal cancer and the advanced adenoma can easily and quickly detect the colorectal cancer and the advanced adenoma at a molecular level, so as to judge the malignancy and prognosis of the colorectal cancer and the advanced adenoma. The diagnostic kit of the present application has high detection efficiency, strong pertinence, and is advantageous in accuracy, reliability, flexibility, rapidity, and low cost, which is beneficial to the early detection and timely treatment of the colorectal cancer and the advanced adenoma.
5. Compared with the traditional colonoscopy screening for colorectal cancer and advanced adenoma, and the conventional fecal occult blood test, the kit provided by the present application adopts methylation-specific PCR method to screen colorectal cancer and advanced adenoma by detecting DNA of fecal exfoliated cells. The kit provided by the present application has the following advantages:
   1) Being completely non-invasive; it is only required to collect 2-5 g of a feces sample, which is highly acceptable for the asymptomatic population;
   2) High accuracy; the kit can detect an absolute majority of colorectal cancer and advanced adenoma, and most precancerous adenomas in preclinical research, can detect 96.3% of early colorectal cancer and 85.8% of precancerous adenomas (≥ 1 cm), and the specificity is 100%;
   3) The tumors on the left and right side colons can be detected at the same time, thus reducing the detection blind spot and avoiding missed diagnosis;
   4) Not only can the colorectal cancer and the advanced adenoma be found, but also the precancerous adenoma can be found, which provides the possibility to prevent the colorectal cancer and the advanced adenoma by removing the precancerous adenoma; and
   5) Easy operation; after obtaining the test kit, the user can complete the collection of samples at home, and then send the collected samples by express to be tested by professionals.
6. This method adopts real-time fluorescence PCR method (Real-time PCR), which is developed based on the common polymerase chain reaction technology. In the process of common polymerase chain reaction amplification, fluorescent dyes or electrophoresis detection are used for quantification, while the application of the probe method of the present application enables the technology to have higher accuracy, higher sensitivity, and higher throughput, and does not require operations such as electrophoresis or hybridization after PCR, thereby reducing contamination and operational errors.

### BRIEF DESCRIPTION OF THE DRAWINGS

The accompanying drawings described herein are used to provide further understanding of the present application and constitute a part of the present application. The exemplary embodiments of the present application and their descriptions are used to explain the present application and do not constitute an improper limitation of the present application. In the drawings:
FIG. 1 shows detection results of PRDM12 methylation;
FIG. 2 shows detection results of FOXE1 methylation;
FIG. 3 shows detection results of B3GAT2 methylation;
FIG. 4 shows detection results of Vimentin methylation;
FIG. 5 shows detection results of SFRP2-1 methylation; and
FIG. 6 shows detection results of SFRP2-2 methylation.

### DETAILED DESCRIPTION OF THE EMBODIMENTS

In order to make the technical problems to be solved, technical solutions, and beneficial effects of the present application clearer and more comprehensible, the present application will be further described in detail below with reference to the accompanying drawings and embodiments. It should be understood that the specific embodiments described herein are only used to explain the present application, but not to limit the present application.

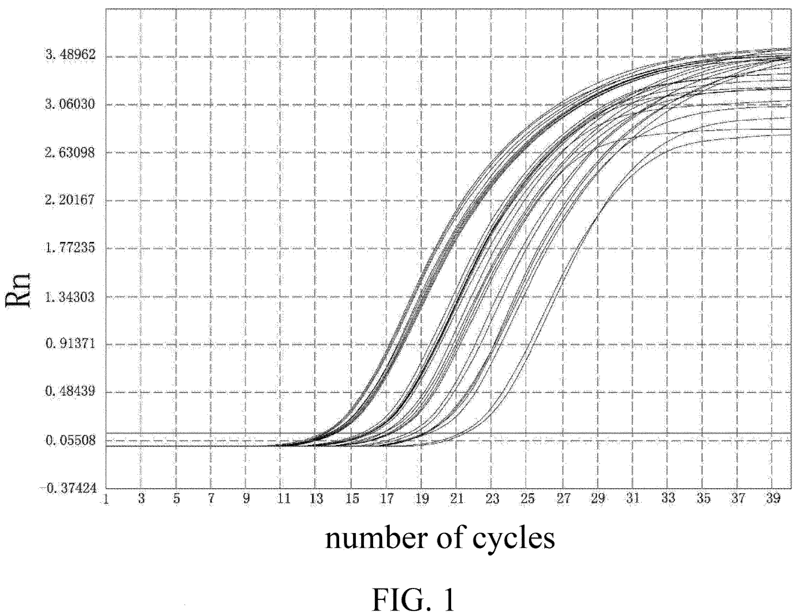

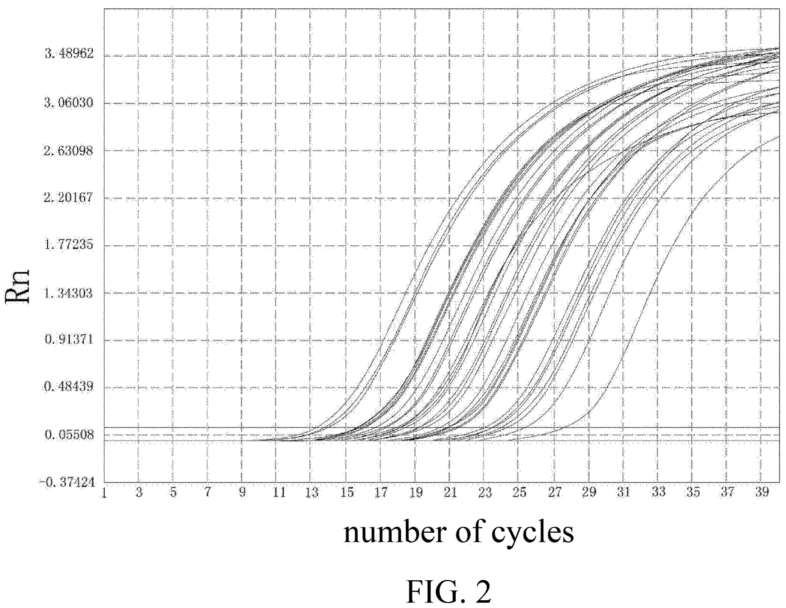

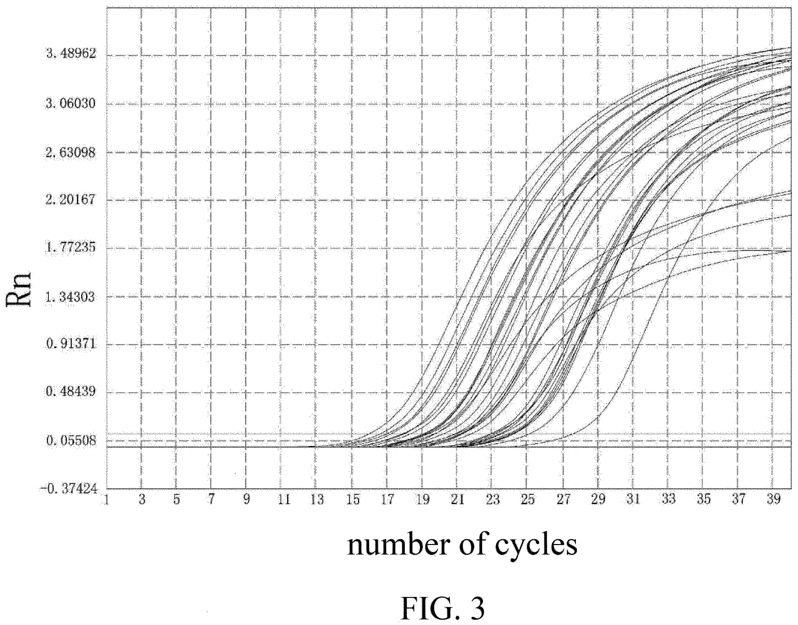

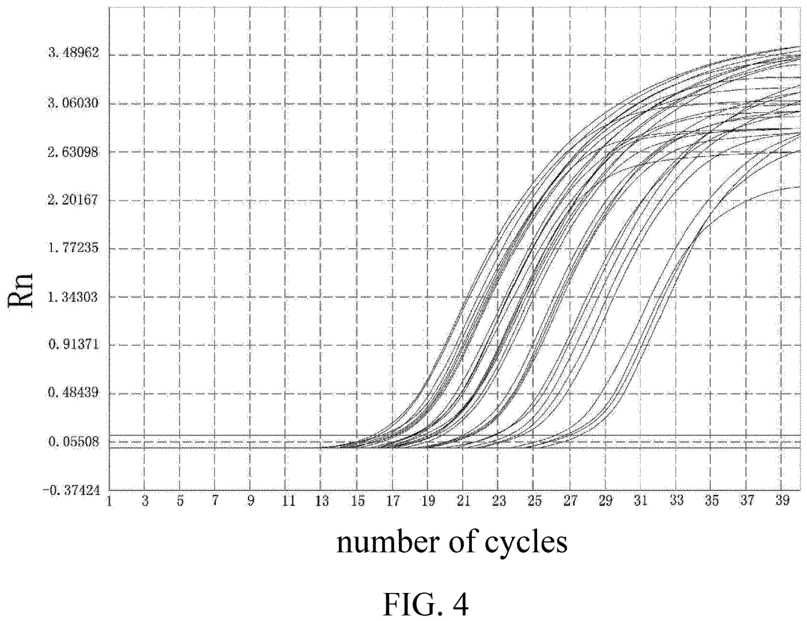

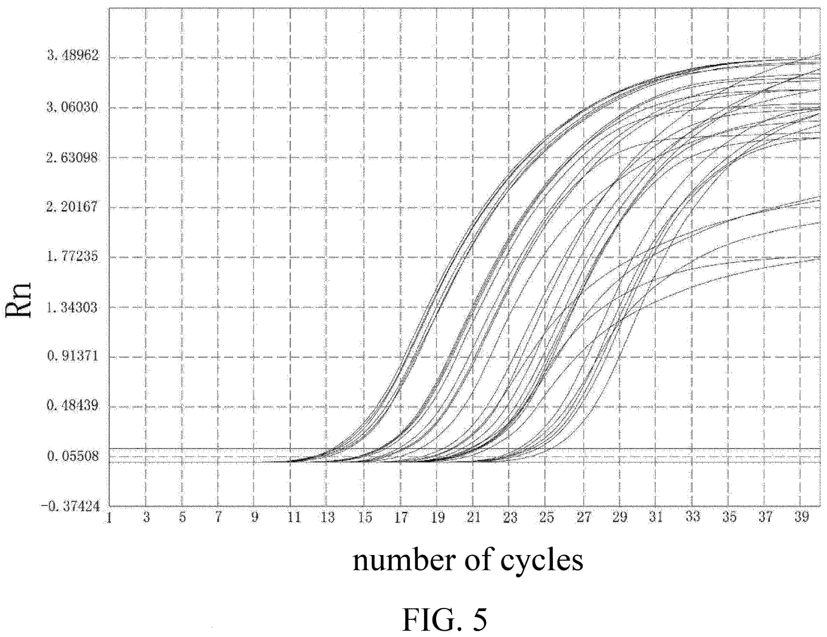

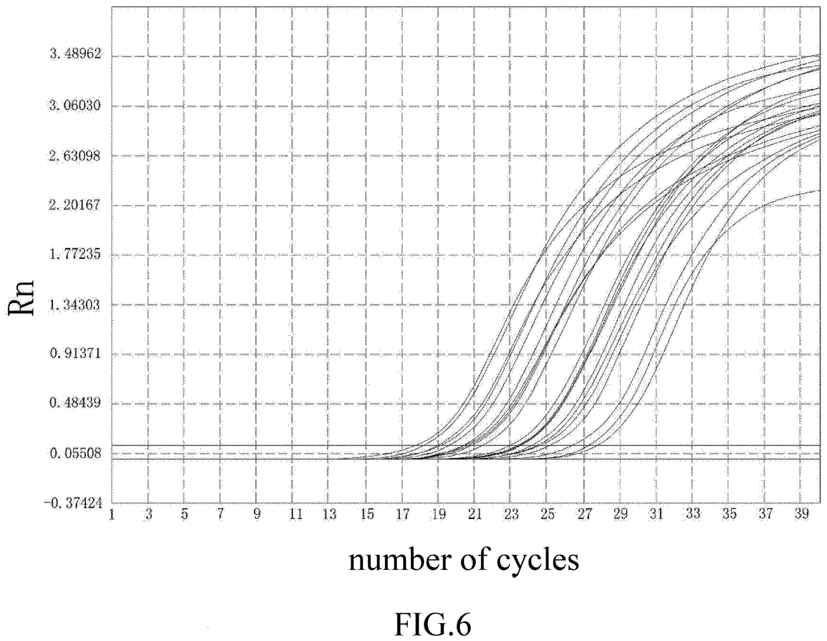

Referring to FIGS. 1-6, the detection results of methylation of related genes (FAM channel) are shown.

The present application is described in further details hereinbelow in conjunction with embodiments.
1. Sample collection from study objects:

In this study, samples were surgically resected from 31 cases of colorectal cancer patients in Shanghai Changhai Hospital were selected as the study objects. All cases were diagnosed based on the patient's clinical data, imaging and colonoscopy, etc., and were confirmed by postoperative pathological diagnosis. The surgically resected colorectal cancer tissue and paracancerous tissue (normal colorectal mucosal tissue with a distance of ≥ 5 cm from the cancer tissue) were snap-frozen in liquid nitrogen and stored in a -80 degree refrigerator. An appropriate amount of the cancer tissue and the paracancerous tissue were collected, and genomic DNA was extracted therefrom.

In this study, 31 colorectal cancer patients and feces samples from corresponding patients from Shanghai Changhai Hospital were selected as research objects. The diagnosis of the cases used was determined based on the clinical data, imaging, colonoscopy, and postoperative pathology of the patients. The collected feces samples were stored in a collection tube containing feces DNA preservation solution and placed in a -20 degree refrigerator. An appropriate amount of each feces sample was collected to extract genomic DNA.

### 2. Extraction of genomic DNA

Tissue samples were extracted from cancer tissues and paracancerous tissues using the QIAamp DNA Mini Kit Tissue, Whole Genome DNA Extraction Kit (Qiagen, Germany). Feces samples were obtained using the QIAamp DNA feces Mini Kit, Feces Genomic DNA Extraction Kit. The concentration and quality of genomic DNA were detected by NanoDrop2000 microspectrophotometer (Thermo Fisher Scientific, USA) for subsequent methylation PCR detection for specific regions of genes. The specific extraction steps were carried out according to the operating instructions of the extraction kit.

### 3. Selection of control groups

The positive control is a mixture of the internal reference ACTB gene and methylated plasmid DNA fragments of PRDM12, FOXE1, B3GAT2, Vimentin, SFRP2-1, and SFRP2-2, or is a positive control of methylated human whole genome DNA. The negative control is a mixture of the internal reference ACTB gene and unmethylated plasmid DNA fragments of PRDM12, FOXE1, B3GAT2, Vimentin, SFRP2-1, and SFRP2-2, or is a negative control of unmethylated human whole genome DNA, or is a negative control of unmethylated human whole genome DNA. The blank control is a Tris-EDTA buffer or nuclease-free water.

### 4. DNA bisulfite conversion

The genomic DNA obtained from feces, blood, or tissues was treated with a DNA bisulfite conversion kit of ZYMO EZ DNA Methylation Gold Kit (ZYMO Company, USA) according to the instructions, in which, the treatment mainly included three steps of sulfonation, hydrolyzation, and deamination, such that unmethylated cytosine (C) was converted to uracil (U), while methylated cytosine (5mC) was unable to be converted. The genomic DNA of the sample to be tested after being converted by bisulfite was used as a template for methylation PCR detection. The positive control and the negative control were genetically engineered plasmids.

### 5. Design and synthesis of primers and probes

For the specific methylation sites of PRDM12, FOXE1, B3GAT2, Vimentin, SFRP2-1, SFRP2-2 genes in the human genome and the internal reference gene ACTB (referring to the sequence of the entire human genome published in the NCBI database), Primer Premier 3.0 and BeaconDesigner software were adopted to design specific primers and probes. Primer and probe were synthesized by Sangon Bioengineering (Shanghai) Co., Ltd.

The present application simultaneously designed and verified multiple sets of primer and probe combination sequences for the methylated nucleic acid sequences of PRDM12, FOXE1, B3GAT2, Vimentin, SFRP2-1, and SFRP2-2 target gene fragments related to colorectal cancer and advanced adenoma. After repeated optimization experiments, the following sequences (Table 1) of primer-probe sets for detecting methylation of PRDM12, FOXE1, B3GAT2, Vimentin, SFRP2-1, and SFRP2-2 gene fragments were optimized. Sequences of specific primer-probe sets are shown in Table 1:

**Table 1 Sequences of specific primer-probe sets for detecting methylation in specific regions of genes**

| Gene name | Primer sequences: forward primer (F), reverse primer (R) | Primer length |
|---|---|---|
| PRDM12 | F: 5'- GTTAGATTAGAAGtATAAAATGTG -3' (primer 1) | 24 nt |
| | R: 5'- ATCCCATTCCTCTCCTCC -3' (primer 2) | 18 nt |
| | P : 5'-AGCGCGGTGGAGATTTG -3' (probe 1) | 17 nt |
| FOXE1 | F: 5'- CGAGTttAAGtttTTGGtAGAGG -3' (primer 3) | 23 nt |
| | R: 5'- CCGATCGACTaAaaCCCGa -3' (primer 4) | 19 nt |
| | P : 5'- tCGAGTTCGGGCGtTGAGG -3' (probe 2) | 19 nt |
| B3GAT2 | F: 5'- TtCGtCGGGTtTGGCG -3' (primer 5) | 16 nt |
| | R: 5'- CAaaAaaTCGTaCAaCCCCG -3' (primer 6) | 20 nt |
| | P : 5'- tCGCGAGtAAGtTCGGGAG -3' (probe 3) | 19 nt |
| Vimentin | F: 5'- GtCGtAGttTtTACGttTCGT -3' (primer 7) | 21 nt |
| | R: 5'-aaCGCACGaCAaAaaAaCG -3' (primer 8) | 19 nt |
| | P : 5'- ttCGGGCGGCGTGTATG -3' (probe 4) | 17 nt |
| SFRP2-1 | F: 5'- CGGAtTGGGGtAAAAtAAGttC -3' (primer 9) | 22 nt |
| | R: 5'- CTaaCGaaAACGCGCCTA -3' (primer 10) | 18 nt |
| | P : 5'- TAGGCGCGTTttCGttAGTAttTGG -3' (probe 5) | 25 nt |
| SFRP2-2 | F: 5'- AATGtAGtCGGCGCG -3' (primer 11) | 15 nt |
| | R: 5'- CCTTaTTaaaAaTTCAAaaAaCCCG -3' (primer 12) | 25 nt |
| | P:5'- AGttAtTTtCGGGCGTGCGGt -3' (probe 6) | 21 nt |
| ACTB | F: 5'- GTGATGGAGGAGGTTTAGTAAG -3' (primer 13) | 22 nt |
| | R: 5'- CAATAAAACCTACTCCTCCCTT -3' (primer 14) | 22 nt |
| | P:5'- TGTGTTTGTTATTGTGTGTTGGGTGGT -3' (probe 7) | 27 nt |

### 6. Components of PCR reaction system

The PCR reaction system includes the above-mentioned specific primers, which can be divided into 6 reaction systems (A/B/C/D/E/F), in which:
the PCR system A comprises: a forward primer (primer 1) for the PRDM12 gene, a reverse primer (primer 2) for the PRDM12 gene, and a detection probe (probe 1) for the PRDM12 gene. The PCR system B comprises: a forward primer (primer 3) for the FOXE1 gene, a reverse primer (primer 4) for the FOXE1 gene, and a detection probe (probe 2) for the FOXE1 gene. The PCR system C comprises: a forward primer (primer 5) for the B3GAT2 gene, a reverse primer (primer 6) for the B3GAT2 gene, and a detection probe (probe 3) for the B3GAT2 gene. The PCR system D comprises: a forward primer (primer 7) for the Vimentin gene, a reverse primer (primer 8) for the Vimentin gene, and a detection probe (probe 4) for the Vimentin gene. The PCR system E comprises: a forward primer (primer 9) for the SFRP2-1 gene, a reverse primer (primer 10) for the SFRP2-1 gene, and a detection probe (probe 5) for the SFRP2-1 gene. The PCR system F comprises: a forward primer (primer 11) for the SFRP2-2 gene, a reverse primer (primer 12) for the SFRP2-2 gene, and a detection probe (probe 6) for the SFRP2-2 gene. The PCR reaction system further comprises: a forward primer (primer 13) for the internal reference gene ACTB, a reverse primer (primer 14) for the internal reference gene ACTB, a detection probe (probe 7) for the ACTB, a PCR buffer, dNTPs, a hot-start taq polymerase, and MgCl₂.

The converted DNA was added to each PCR reaction system, and the final concentration of each component was shown in Table 2:

**Table 2 Components of the reaction system**

| Rection system A/B/C/D/E/F | | |
|---|---|---|
| Reagent | specification | Volume (µL) |
| PCR buffer | / | 2.5 |
| 25mM MgCl₂ | 25 mM | 4 |
| 100mM dATP | 100 mM | 0.05 |
| 100mM dTTP | 100 mM | 0.05 |
| 100mM dCTP | 100 mM | 0.05 |
| 100mM dGTP | 100 mM | 0.05 |
| hot-start taq polymerase | 5 U/µL | 0.3 |
| probe | 50 µM | 0.1 |
| primer | 50 µM | 0.2 |
| primer | 50 µM | 0.2 |
| nuclease-free water | | 22.5 |
| Converted DNA template | 10 | |
| Total volume | 40 | |

### 7. Use of the kit, which included the following steps:

(1) Samples to be tested were prepared, in which, feces, blood, or tissue-derived samples were all applicable to the present application. Genomic DNA was extracted from the feces, blood, or tissue samples and converted by bisulfite, and the converted DNA was used as a template for PCR reactions.
(2) The PCR reaction reagent was taken from the kit, equilibrated to room temperature, 29.7 µL × (n+1), PCR reaction solution and 0.3 µL × (n+1) enzyme mixture were uniformly mixed for 20 s, and centrifuged instantaneously; in which, n = number of samples tested, instantaneous centrifugation was performed.
(3) a resulting centrifuged solution was dispensed into PCR tubes with each tube containing 30 µL of the centrifuged solution.
(4) 10 µL of a template DNA solution (converted DNA to be tested or converted negative/positive/the blank control) was added into each PCR tube with a micropipette, to make a final volume of each reaction be 40 µL. A tube cap was immediately closed, and the contents in the tube were mixed uniformly, centrifuged instantaneously, and transferred the PCR tube to the real-time fluorescence quantitative PCR amplifier.
(5) In the real-time fluorescence quantitative PCR instrument, an amplification program was set to continuously complete PCR amplification and real-time fluorescence signal acquisition and analysis during the amplification process. The reaction program settings are shown in Table 3:

**Table 3**

| stages | cycle number | reaction temperature | reaction time |
|---|---|---|---|
| first stage | 1 | 95.0°C | 1 min |
| second stage | 10 | 95.0°C | 20 s |
| | | 65.0°C | 30 s (-0.5°C/Cycler) |
| | | 72.0°C | 20 s |
| third stage | 35 | 95.0°C | 20 s |
| | | 60.0°C | 26 s (FAM, HEX, ROX, and Cy5 are lightened, and fluorescence value is detected once before the end of each cycle) |
| | | 72.0°C | 30 s |

(6) determination of PCR results

During the sample detection, the baseline was determined by taking fluorescence signals of between 3 and 15 cycles; a threshold was set by making a threshold line exceeding a highest point of an amplification curve of a normal negative control, and the Ct value was Undet; positive or negative result of the sample was determined according to reported Ct values, that is, according to a cycle number experienced when the fluorescent signal in each reaction tube reaches the set threshold.

Conclusions: (1) 31 positive samples have amplification curves in the corresponding detection wells of FAM channel, and the detection results are positive, which are consistent with the pathological diagnosis results. In the 10 negative samples, only the internal reference gene ROX channel of the control has an amplification curve, and the results of the detection of the target gene in the FAM channel are all negative, which are consistent with the pathological diagnosis results. It shows that the detection results of the kit provided by the present application have extremely high specificity and reliability. The Ct values of the detailed detection results of each sample of the present application are shown in Table 3.

It can be seen from the fluorescence quantitative PCR detection results of the samples that the PRDM12, FOXE1, B3GAT2, Vimentin, SFRP2-1, and SFRP2-2 genes of each sample are highly amplified in colorectal cancer and advanced adenoma samples, while the amplification in normal samples is extremely low. Therefore, it is proved that the kit of the present application can rapidly and accurately detect mutant genes and normal genes in feces exfoliated cells from colorectal cancer patient and advanced adenoma patient. It can be seen that the kit of the present application is not only fast and efficient, but also the interpretation of the results is very clear and intuitive, and the results are reliable and specific.

Table 3 Ct value of each sample fluorescence quantitative PCR detection result (in NO. "1-N", "1-P", and "1-F", "1"represents the sample number, "N" represents a normal tissue, "P" represents a cancer or adenoma tissue, and "F" represents a feces sample , and so on)

| NO. | ACTB internal reference) | PRDM12 | result | NO. | ACTB (internal reference) | PRDM12 | result | NO. | ACTB (internal reference) | PRDM12 | result |
|---|---|---|---|---|---|---|---|---|---|---|---|
| 1-N | 15.7 | undet | negative | 1-P | 14.7 | 18.9 | positive | 1-F | 18 18.8 | 26.3 | positive |
| 2-N | 16.9 | undet | negative | 2-P | 15.3 | 19.3 | positive | 2-F | 17.9 | 23.4 | positive |
| 3-N | 15.2 | undet | negative | 3-P | 19.2 | 15.5 | positive | 3-F | 24 .4 | 25.4 | positive |
| 4-N | 16.3 | undet | negative | 4-P | 20.1 | 25.5 | positive | 4-F | 23 .1 | 27.7 | positive |
| 5-N | 17.9 | undet | negative | 5-P | 14.3 | 20.5 | positive | 5-F | 14 .7 | 18.3 | positive |
| 6-N | 18.4 | undet | negative | 6-P | 16.7 | 25.4 | positive | 6-F | 18 .8 | 25.7 | positive |
| 7-N | 19.3 | undet | negative | 7-P | 16.3 | 23.9 | positive | 7-F | 19 .2 | 28.6 | positive |
| 8-N | 16.8 | undet | negative | 8-P | 16.1 | 25.2 | positive | 8-F | 20 0.1 | 23.4 | positive |
| 9-N | 17.9 | undet | negative | 9-P | 14.5 | 22.1 | positive | 9-F | 25 .3 | undet | negative |
| 10-N | 18.8 | undet | negative | 10-P | 16.3 | 23.9 | positive | 10-F | 23 .1 | 28.6 | positive |
| 11-N | 19.2 | undet | negative | 11-P | 18.8 | 24.3 | positive | 11-F | 25 .8 | undet | negative |
| 12-N | 21.4 | undet | negative | 12-P | 21.4 | 25.5 | positive | 12-F | 18 .8 | 24.3 | positive |
| 13-N | 15.3 | undet | negative | 13-P | 21.3 | 27.3 | positive | 13-F | 19 .2 | 23.4 | positive |
| 14-N | 16.7 | undet | negative | 14-P | 17.1 | 28.4 | positive | 14-F | 20 .1 | 27.4 | positive |
| 15-N | 13.9 | undet | negative | 15-P | 14.7 | 18.8 | positive | 15-F | 16 .3 | 23.2 | positive |
| 16-N | 14.4 | undet | negative | 16-P | 14.4 | 19.6 | positive | 16-F | 18 .8 | 27.1 | positive |
| 17-N | 16.3 | undet | negative | 17-P | 13.1 | 22.9 | positive | 17-F | 23 .4 | 26.4 | positive |
| 18-N | 16.8 | undet | negative | 18-P | 14.7 | 21.1 | positive | 18-F | 21 .3 | 26.1 | positive |
| 19-N | 21.9 | undet | negative | 19-P | 18.8 | 18.3 | positive | 19-F | 25 .8 | undet | negative |
| 20-N | 20.8 | undet | negative | 20-P | 19.2 | 18.9 | positive | 20-F | 24 .7 | 25.1 | positive |
| 21-N | 17.7 | undet | negative | 21-P | 20.1 | 23.9 | positive | 21- F | 24.4 | 25.3 | positive |
| 22-N | 14.5 | undet | negative | 22-P | 15.3 | 21.7 | positive | 22-F | 23.1 | 26.5 | positive |
| 23-N | 16.3 | undet | negative | 23-P | 16.7 | 23.8 | positive | 23-F | 18.9 | 19.9 | positive |
| 24-N | 18.8 | undet | negative | 24-P | 19.2 | 25.4 | positive | 24-F | 18.8 | 26.6 | positive |
| 25-N | 19.2 | undet | negative | 25-P | 20.1 | 24.7 | positive | 25-F | 19.2 | 27.3 | positive |
| 26-N | 15.5 | undet | negative | 26-P | 14.3 | 21.7 | positive | 26-F | 20.1 | 25.4 | positive |
| 27-N | 17.3 | undet | negative | 27-P | 16.7 | 23.3 | positive | 27-F | 25.3 | undet | negative |
| 28-N | 18.8 | undet | negative | 28-P | 17.8 | 20.5 | positive | 28-F | 18.8 | 25.4 | positive |
| 29-N | 19.2 | undet | negative | 29-P | 17.2 | 21.7 | positive | 29-F | 23.5 | 25.4 | positive |
| 30-N | 14.4 | undet | negative | 30-P | 13.4 | 17.6 | positive | 30-F | 26.1 | undet | negative |
| 31-N | 13.1 | undet | negative | 31-P | 11.3 | 15.5 | positive | 31-F | 25.4 | 28.8 | positive |

| NO. al | ACTB (intern refere nce) | FOXE1 | result | NO. | (intern refere nce) | ACTB FOXE 1 a1 | result | NO. | ACTB (intern a1 refere nce) | FOXE 1 | result |
|---|---|---|---|---|---|---|---|---|---|---|---|
| 1-N | 15.7 | undet | negative | 1-P | 14.7 | 17.6 | positive | 1-F | 18.8 | 21.7 | positive |
| 2-N | 16.9 | undet | negative | 2-P | 15.3 | 17.6 | positive | 2-F | 17.9 | 26.1 | positive |
| 3-N | 15.2 | undet | negative | 3-P | 19.2 | 25.9 | positive | 3-F | 24.4 | 25.1 | positive |
| 4-N | 16.3 | undet | negative | 4-P | 20.1 | 18.4 | positive | 4-F | 23.1 | 20.5 | positive |
| 5-N | 17.9 | undet | negative | 5-P | 14.3 | 21.5 | positive | 5-F | 14.7 | 21.7 | positive |
| 6-N | 18.4 | undet | negative | 6-P | 16.7 | 17.3 | positive | 6-F | 18.8 | 23.9 | positive |
| 7-N | 19.3 | undet | negative | 7-P | 16.3 | 20.5 | positive | 7-F | 19.2 | 26.1 | positive |
| 8-N | 16.8 | undet | negative | 8-P | 16.1 | 25.4 | positive | 8-F | 20.1 | 20.5 | positive |
| 9-N | 17.9 | undet | negative | 9-P | 14.5 | 23.9 | positive | 9-F | 25.3 | 21.7 | positive |
| 10-N | 18.8 | undet | negative | 10-P | 16.3 | 25.2 | positive | 10-F | 23.1 | 23.4 | positive |
| 11-N | 19.2 | undet | negative | 11-P | 18.8 | 22.1 | positive | 11-F | 25.8 | 25.4 | positive |
| 12-N | 21.4 | undet | negative | 12-P | 21.4 | 25.4 | positive | 12-F | 18.8 | 25.4 | positive |
| 13-N | 15.3 | undet | negative | 13-P | 21.3 | 21.7 | positive | 13-F | 19.2 | 26.1 | positive |
| 14-N | 16.7 | undet | negative | 14-P | 17.1 | 17.6 | positive | 14-F | 20.1 | 21.7 | positive |
| 15-N | 13.9 | undet | negative | 15-P | 14.7 | 21.1 | positive | 15-F | 16.3 | 20.4 | positive |
| 16-N | 14.4 | undet | negative | 16-P | 14.4 | 17.6 | positive | 16-F | 18.8 | 23.9 | positive |
| 17-N | 16.3 | undet | negative | 17-P | 13.1 | 19.4 | positive | 17-F | 23.4 | 27.7 | positive |
| 18-N | 16.8 | undet | negative | 18-P | 14.7 | 23.9 | positive | 18-F | 21.3 | 28.5 | positive |
| 19-N | 21.9 | undet | negative | 19-P | 18.8 | 17.3 | positive | 19-F | 25.8 | 25.2 | positive |
| 20-N | 20.8 | undet | negative | 20-P | 19.2 | 27.1 | positive | 20-F | 24.7 | 26.1 | positive |
| 21-N | 17.7 | undet | negative | 21-P | 20.1 | 21.7 | positive | 21-F | 24.4 | 21.7 | positive |
| 22-N | 14.5 | undet | negative | 22-P | 15.3 | 22.5 | positive | 22-F | 23.1 | 24.8 | positive |
| 23-N | 16.3 | undet | negative | 23-P | 16.7 | 26.2 | positive | 23-F | 18.9 | 26.1 | positive |
| 24-N | 18.8 | undet | negative | 24-P | 19.2 | 17.6 | positive | 24-F | 18.8 | 25.4 | positive |
| 25-N | 19.2 | undet | negative | 25-P | 20.1 | 26.2 | positive | 25-F | 19.2 | 26.1 | positive |
| 26-N | 15.5 | undet | negative | 26-P | 14.3 | 20.5 | positive | 26-F | 20.1 | 21.7 | positive |
| 27-N | 17.3 | undet | negative | 27-P | 16.7 | 23.9 | positive | 27-F | 25.3 | undet | negative |
| 28-N | 18.8 | undet | negative | 28-P | 17.8 | 22.9 | positive | 28-F | 18.8 | 25.1 | positive |
| 29-N | 19.2 | undet | negative | 29-P | 17.2 | 23.7 | positive | 29-F | 23.5 | undet | negative |
| 30-N | 14.4 | undet | negative | 30-P | 13.4 | 20.5 | positive | 30-F | 26.1 | 30.1 | positive |
| 31-N | 13.1 | undet | negative | 31-P | 11.3 | 14.3 | positive | 31-F | 25.4 | 26.8 | positive |

| NO. a1 | ACTB refere nce) | intern 2 B3GAT | result | NO. | ACTB (intern al refere nce) | B3GA T2 | result | NO. | ACTB (intern T2 al refere nce) | B3GA | result |
|---|---|---|---|---|---|---|---|---|---|---|---|
| 1-N | 15.7 | undet | negative | 1-P | 14.7 | 18.9 | positive | 1-F | 18.8 | 27.2 | positive |
| 2-N | 16.9 | undet | negative | 2-P | 15.3 | 18.9 | positive | 2-F | 17.9 | 27.7 | positive |
| 3-N | 15.2 | undet | negative | 3-P | 19.2 | 21.5 | positive | 3-F | 24.4 | 26.1 | positive |
| 4-N | 16.3 | undet | negative | 4-P | 20.1 | 27.1 | positive | 4-F | 23.1 | 26.8 | positive |
| 5-N | 17.9 | undet | negative | 5-P | 14.3 | 17.6 | positive | 5-F | 14.7 | 23.4 | positive |
| 6-N | 18.4 | undet | negative | 6-P | 16.7 | 24.3 | positive | 6-F | 18.8 | 21.7 | positive |
| 7-N | 19.3 | undet | negative | 7-P | 16.3 | 21.1 | positive | 7-F | 19.2 | 27.4 | positive |
| 8-N | 16.8 | undet | negative | 8-P | 16.1 | 18.3 | positive | 8-F | 20.1 | 25.4 | positive |
| 9-N | 17.9 | undet | negative | 9-P | 14.5 | 18.9 | positive | 9-F | 25.3 | 25.1 | positive |
| 10-N | 18.8 | undet | negative | 10-P | 16.3 | 23.9 | positive | 10-F | 23.1 | 26.1 | positive |
| 11-N | 19.2 | undet | negative | 11-P | 18.8 | 27.2 | positive | 11-F | 25.8 | 21.7 | positive |
| 12-N | 21.4 | undet | negative | 12-P | 21.4 | 18.3 | positive | 12-F | 18.8 | 27.1 | positive |
| 13-N | 15.3 | undet | negative | 13-P | 21.3 | 19.3 | positive | 13-F | 19.2 | 27.7 | positive |
| 14-N | 16.7 | undet | negative | 14-P | 17.1 | 20.5 | positive | 14-F | 20.1 | 27.1 | positive |
| 15-N | 13.9 | undet | negative | 15-P | 14.7 | 19.6 | positive | 15-F | 16.3 | 21.8 | positive |
| 16-N | 14.4 | undet | negative | 16-P | 14.4 | 17.6 | positive | 16-F | 18.8 | 25.3 | positive |
| 17-N | 16.3 | undet | negative | 17-P | 13.1 | 19.1 | positive | 17-F | 23.4 | 24.4 | positive |
| 18-N | 16.8 | undet | negative | 18-P | 14.7 | 17.6 | positive | 18-F | 21.3 | 25.4 | positive |
| 19-N | 21.9 | undet | negative | 19-P | 18.8 | 19.3 | positive | 19-F | 25.8 | 29.2 | positive |
| 20-N | 20.8 | undet | negative | 20-P | 19.2 | 15.5 | positive | 20-F | 24.7 | 25.6 | positive |
| 21-N | 17.7 | undet | negative | 21-P | 20.1 | 25.5 | positive | 21-F | 24.4 | 25.1 | positive |
| 22-N | 14.5 | undet | negative | 22-P | 15.3 | 21.8 | positive | 22-F | 23.1 | 28.8 | positive |
| 23-N | 16.3 | undet | negative | 23-P | 16.7 | 21.7 | positive | 23-F | 18.9 | 21.7 | positive |
| 24-N | 18.8 | undet | negative | 24-P | 19.2 | 23.2 | positive | 24-F | 18.8 | 27.1 | positive |
| 25-N | 19.2 | undet | negative | 25-P | 20.1 | 21.8 | positive | 25-F | 19.2 | 25.6 | positive |
| 26-N | 15.5 | undet | negative | 26-P | 14.3 | 17.6 | positive | 26-F | 20.1 | 27.3 | positive |
| 27-N | 17.3 | undet | negative | 27-P | 16.7 | 22.8 | positive | 27-F | 25.3 | undet | negative |
| 28-N | 18.8 | undet | negative | 28-P | 17.8 | 21.7 | positive | 28-F | 18.8 | 26.1 | positive |
| 29-N | 19.2 | undet | negative | 29-P | 17.2 | 21.1 | positive | 29-F | 23.5 | undet | negative |
| 30-N | 14.4 | undet | negative | 30-P | 13.4 | 19.3 | positive | 30-F | 26.1 | 28.2 | positive |
| 31-N | 13.1 | undet | negative | 31-P | 11.3 | 23.9 | positive | 31-F | 25.4 | 25.1 | positive |

| NO. al | ACTB (intern refere nce) | Vimenti n | result | NO. | ACTB (intern a1 refere nce) | Vimen tin | result | NO. | ACTB (intern refere nce) | Vimen tin a1. | result |
|---|---|---|---|---|---|---|---|---|---|---|---|
| 1-N | 15.7 | undet | negative | 1-P | 14.7 | 17.3 | positive | 1-F | 18.8 | 25.4 | positive |
| 2-N | 16.9 | undet | negative | 2-P | 15.3 | 17.3 | positive | 2-F | 17.9 | 23.9 | positive |
| 3-N | 15.2 | undet | negative | 3-P | 19.2 | 16.3 | positive | 3-F | 24.4 | 26.4 | positive |
| 4-N | 16.3 | undet | negative | 4-P | 20.1 | 17.6 | positive | 4-F | 23.1 | 21.7 | positive |
| 5-N | 17.9 | undet | negative | 5-P | 14.3 | 21.8 | positive | 5-F | 14.7 | 23.9 | positive |
| 6-N | 18.4 | undet | negative | 6-P | 16.7 | 21.1 | positive | 6-F | 18.8 | 25.4 | positive |
| 7-N | 19.3 | undet | negative | 7-P | 16.3 | 18.3 | positive | 7-F | 19.2 | 24.3 | positive |
| 8-N | 16.8 | undet | negative | 8-P | 16.1 | 19.3 | positive | 8-F | 20.1 | 25.4 | positive |
| 9-N | 17.9 | undet | negative | 9-P | 14.5 | 15.5 | positive | 9-F | 25.3 | undet | negative |
| 10-N | 18.8 | undet | negative | 10-P | 16.3 | 25.5 | positive | 10-F | 23.1 | 21.7 | positive |
| 11-N | 19.2 | undet | negative | 11-P | 18.8 | 20.5 | positive | 11-F | 25.8 | 23.4 | positive |
| 12-N | 21.4 | undet | negative | 12-P | 21.4 | 25.4 | positive | 12-F | 18.8 | 25.4 | positive |
| 13-N | 15.3 | undet | negative | 13-P | 21.3 | 23.9 | positive | 13-F | 19.2 | 24.4 | positive |
| 14-N | 16.7 | undet | negative | 14-P | 17.1 | 25.2 | positive | 14-F | 20.1 | 25.2 | positive |
| 15-N | 13.9 | undet | negative | 15-P | 14.7 | 22.1 | positive | 15-F | 16.3 | 25.4 | positive |
| 16-N | 14.4 | undet | negative | 16-P | 14.4 | 18.2 | positive | 16-F | 18.8 | 23.9 | positive |
| 17-N | 16.3 | undet | negative | 17-P | 13.1 | 20.5 | positive | 17-F | 23.4 | 26.1 | positive |
| 18-N | 16.8 | undet | negative | 18-P | 14.7 | 20.5 | positive | 18-F | 21.3 | 24.9 | positive |
| 19-N | 21.9 | undet | negative | 19-P | 18.8 | 25.1 | positive | 19-F | 25.8 | 25.1 | positive |
| 20-N | 20.8 | undet | negative | 20-P | 19.2 | 26.6 | positive | 20-F | 24.7 | 25.6 | positive |
| 21-N | 17.7 | undet | negative | 21-P | 20.1 | 25.7 | positive | 21-F | 24.4 | 26.1 | positive |
| 22-N | 14.5 | undet | negative | 22-P | 15.3 | 23.9 | positive | 22-F | 23.1 | 25.2 | positive |
| 23-N | 16.3 | undet | negative | 23-P | 16.7 | 24.7 | positive | 23-F | 18.9 | 23.4 | positive |
| 24-N | 18.8 | undet | negative | 24-P | 19.2 | 23.9 | positive | 24-F | 18.8 | 26.6 | positive |
| 25-N | 19.2 | undet | negative | 25-P | 20.1 | 26.3 | positive | 25-F | 19.2 | 25.4 | positive |
| 26-N | 15.5 | undet | negative | 26-P | 14.3 | 21.7 | positive | 26-F | 20.1 | 25.4 | positive |
| 27-N | 17.3 | undet | negative | 27-P | 16.7 | 23.6 | positive | 27-F | 25.3 | undet | negative |
| 28-N | 18.8 | undet | negative | 28-P | 17.8 | 26.1 | positive | 28-F | 18.8 | 24.9 | positive |
| 29-N | 19.2 | undet | negative | 29-P | 17.2 | 21.7 | positive | 29-F | 23.5 | 27.1 | positive |
| 30-N | 14.4 | undet | negative | 30-P | 13.4 | 19.9 | positive | 30-F | 26.1 | 26.3 | positive |
| 31-N | 13.1 | undet | negative | 31-P | 11.3 | 19.3 | positive | 31-F | 25.4 | 26.1 | positive |

| NO. al | ACTB (intern refere nce) | SFRP2-1 | result | NO. | ACTB (intern 2-1 2 al refere nce) | SFRP | result | NO. | ACTB (intern a1 refere nce) | SFRP 2-1 | result |
|---|---|---|---|---|---|---|---|---|---|---|---|
| 1-N | 15.7 | undet | negative | 1-P | 14.7 | 21.1 | positive | 1-F | 18.8 | 20.5 | positive |
| 2-N | 16.9 | undet | negative | 2-P | 15.3 | 21.1 | positive | 2-F | 17.9 | 25.4 | positive |
| 3-N | 15.2 | undet | negative | 3-P | 19.2 | 22.1 | positive | 3-F | 24.4 | 23.1 | positive |
| 4-N | 16.3 | undet | negative | 4-P | 20.1 | 24.5 | positive | 4-F | 23.1 | 25.4 | positive |
| 5-N | 17.9 | undet | negative | 5-P | 14.3 | 18.9 | positive | 5-F | 14.7 | 23.9 | positive |
| 6-N | 18.4 | undet | negative | 6-P | 16.7 | 22.5 | positive | 6-F | 18.8 | 25.1 | positive |
| 7-N | 19.3 | undet | negative | 7-P | 16.3 | 19.3 | positive | 7-F | 19.2 | 21.7 | positive |
| 8-N | 16.8 | undet | negative | 8-P | 16.1 | 17.6 | positive | 8-F | 20.1 | 28.8 | positive |
| 9-N | 17.9 | undet | negative | 9-P | 14.5 | 21.8 | positive | 9-F | 25.3 | 21.7 | positive |
| 10-N | 18.8 | undet | negative | 10-P | 16.3 | 21.1 | positive | 10-F | 23.1 | 27.1 | positive |
| 11-N | 19.2 | undet | negative | 11-P | 18.8 | 18.3 | positive | 11-F | 25.8 | 28.1 | positive |
| 12-N | 21.4 | undet | negative | 12-P | 21.4 | 21.7 | positive | 12-F | 18.8 | 21.7 | positive |
| 13-N | 15.3 | undet | negative | 13-P | 21.3 | 15.5 | positive | 13-F | 19.2 | 25.1 | positive |
| 14-N | 16.7 | undet | negative | 14-P | 17.1 | 25.5 | positive | 14-F | 20.1 | 20.5 | positive |
| 15-N | 13.9 | undet | negative | 15-P | 14.7 | 20.5 | positive | 15-F | 16.3 | 20.2 | positive |
| 16-N | 14.4 | undet | negative | 16-P | 14.4 | 25.4 | positive | 16-F | 18.8 | 21.7 | positive |
| 17-N | 16.3 | undet | negative | 17-P | 13.1 | 16.5 | positive | 17-F | 23.4 | 21.7 | positive |
| 18-N | 16.8 | undet | negative | 18-P | 14.7 | 21.7 | positive | 18-F | 21.3 | 27.2 | positive |
| 19-N | 21.9 | undet | negative | 19-P | 18.8 | 20.2 | positive | 19-F | 25.8 | 26.1 | positive |
| 20-N | 20.8 | undet | negative | 20-P | 19.2 | 26.3 | positive | 20-F | 24.7 | undet | negative |
| 21-N | 17.7 | undet | negative | 21-P | 20.1 | 26.3 | positive | 21-F | 24.4 | 25.1 | positive |
| 22-N | 14.5 | undet | negative | 22-P | 15.3 | 20.6 | positive | 22-F | 23.1 | 26.1 | positive |
| 23-N | 16.3 | undet | negative | 23-P | 16.7 | 21.7 | positive | 23-F | 18.9 | 28.1 | positive |
| 24-N | 18.8 | undet | negative | 24-P | 19.2 | 28.3 | positive | 24-F | 18.8 | 25.1 | positive |
| 25-N | 19.2 | undet | negative | 25-P | 20.1 | 25.5 | positive | 25-F | 19.2 | 26.3 | positive |
| 26-N | 15.5 | undet | negative | 26-P | 14.3 | 25.4 | positive | 26-F | 20.1 | 25.1 | positive |
| 27-N | 17.3 | undet | negative | 27-P | 16.7 | 21.1 | positive | 27-F | 25.3 | 25.4 | positive |
| 28-N | 18.8 | undet | negative | 28-P | 17.8 | 18.3 | positive | 28-F | 18.8 | 25.4 | positive |
| 29-N | 19.2 | undet | negative | 29-P | 17.2 | 25.4 | positive | 29-F | 23.5 | undet | negative |
| 30-N | 14.4 | undet | negative | 30-P | 13.4 | 15.5 | positive | 30-F | 26.1 | 28.1 | positive |
| 31-N | 13.1 | undet | negative | 31-P | 11.3 | 13.2 | positive | 31-F | 25.4 | 27.2 | positive |

| NO. | ACTB al refere nce) | SFRP2-(intern 2 | result | NO. | ACTB (intern 2-2 al refere nce) | SFRP | result | NO. | ACTB (intern al refere nce) | SFRP 2-2 | result |
|---|---|---|---|---|---|---|---|---|---|---|---|
| 1-N | 15.7 | undet | negative | 1-P | 14.7 | 21.9 | positive | 1-F | 18.8 | 20.9 | positive |
| 2-N | 16.9 | undet | negative | 2-P | 15.3 | 17.3 | positive | 2-F | 17.9 | 23.9 | positive |
| 3-N | 15.2 | undet | negative | 3-P | 19.2 | 20.8 | positive | 3-F | 24.4 | 25.4 | positive |
| 4-N | 16.3 | undet | negative | 4-P | 20.1 | 26.9 | positive | 4-F | 23.1 | 27.1 | positive |
| 5-N | 17.9 | undet | negative | 5-P | 14.3 | 16.8 | positive | 5-F | 14.7 | 23.6 | positive |
| 6-N | 18.4 | undet | negative | 6-P | 16.7 | 17.3 | positive | 6-F | 18.8 | 26.1 | positive |
| 7-N | 19.3 | undet | negative | 7-P | 16.3 | 25.4 | positive | 7-F | 19.2 | 28.8 | positive |
| 8-N | 16.8 | undet | negative | 8-P | 16.1 | 23.9 | positive | 8-F | 20.1 | 21.7 | positive |
| 9-N | 17.9 | undet | negative | 9-P | 14.5 | 15.3 | positive | 9-F | 25.3 | undet | negative |
| 10-N | 18.8 | undet | negative | 10-P | 16.3 | 16.7 | positive | 10-F | 23.1 | 26.5 | positive |
| 11-N | 19.2 | undet | negative | 11-P | 18.8 | 19.2 | positive | 11-F | 25.8 | 26.3 | positive |
| 12-N | 21.4 | undet | negative | 12-P | 21.4 | 20.1 | positive | 12-F | 18.8 | 23.9 | positive |
| 13-N | 15.3 | undet | negative | 13-P | 21.3 | 14.3 | positive | 13-F | 19.2 | 26.1 | positive |
| 14-N | 16.7 | undet | negative | 14-P | 17.1 | 16.7 | positive | 14-F | 20.1 | 25.8 | positive |
| 15-N | 13.9 | undet | negative | 15-P | 14.7 | 17.8 | positive | 15-F | 16.3 | 25.3 | positive |
| 16-N | 14.4 | undet | negative | 16-P | 14.4 | 21.9 | positive | 16-F | 18.8 | 23.4 | positive |
| 17-N | 16.3 | undet | negative | 17-P | 13.1 | 17.6 | positive | 17-F | 23.4 | 23.4 | positive |
| 18-N | 16.8 | undet | negative | 18-P | 14.7 | 22.5 | positive | 18-F | 21.3 | 25.8 | positive |
| 19-N | 21.9 | undet | negative | 19-P | 18.8 | 21.9 | positive | 19-F | 25.8 | 29.3 | positive |
| 20-N | 20.8 | undet | negative | 20-P | 19.2 | 28.1 | positive | 20-F | 24.7 | 25.4 | positive |
| 21-N | 17.7 | undet | negative | 21-P | 20.1 | 27.6 | positive | 21-F | 24.4 | 26.1 | positive |
| 22-N | 14.5 | undet | negative | 22-P | 15.3 | 20.5 | positive | 22-F | 23.1 | 24.7 | positive |
| 23-N | 16.3 | undet | negative | 23-P | 16.7 | 25.9 | positive | 23-F | 18.9 | 26.5 | positive |
| 24-N | 18.8 | undet | negative | 24-P | 19.2 | 25.7 | positive | 24-F | 18.8 | 26.1 | positive |
| 25-N | 19.2 | undet | negative | 25-P | 20.1 | 21.7 | positive | 25-F | 19.2 | 27.4 | positive |
| 26-N | 15.5 | undet | negative | 26-P | 14.3 | 21.2 | positive | 26-F | 20.1 | 26.1 | positive |
| 27-N | 17.3 | undet | negative | 27-P | 16.7 | 16.8 | positive | 27-F | 25.3 | undet | negative |
| 28-N | 18.8 | undet | negative | 28-P | 17.8 | 17.3 | positive | 28-F | 18.8 | 25.1 | positive |
| 29-N | 19.2 | undet | negative | 29-P | 17.2 | 25.4 | positive | 29-F | 23.5 | 28.2 | positive |
| 30-N | 14.4 | undet | negative | 30-P | 13.4 | 23.9 | positive | 30-F | 26.1 | 27.5 | positive |
| 31-N | 13.1 | undet | negative | 31-P | 11.3 | 16.3 | positive | 31-F | 25.4 | 26.3 | positive |

The foregoing specification illustrates and describes preferred embodiments of the present application, and as previously stated, it should be understood that the present application is not limited to the form disclosed herein, or should not be construed as an exclusion of other embodiments, but can be used in a variety of other combinations, modifications, and environments. It can be understood that the present application can be modified within the scope of the inventive concepts described herein based on the above teaching and skills or knowledges in relevant fields. However, modifications and changes made by those skilled in the art do not depart from the spirit and scope of the present application, and should all fall within the protection scope of the appended claims of the present application.

## Claims

1. A primer set for detecting methylation of specific regions of human colorectal cancer-related genes, the primer set comprising:
a primer set for methylation areas of any one gene selected from the group consisting of PRDM12, FOXE1, B3GAT2, Vimentin, SFRP2-1, and SFRP2-2, or any gene combination thereof.

2. The primer set for methylation of specific regions of human colorectal cancer-related genes according to claim 1, wherein
a detection primer set for the PRDM12 gene has a specific primer-probe set having the following sequences:
a forward primer: 5'-GTTAGATTAGAAGTATAAAATGTG-3', referred to as a primer 1;
a reverse primer: 5'-ATCCCATTCCTCTCCTCC-3', referred to as a primer 2; and
a detection probe: 5'-AGCGCGGTGGAGATTTG-3', referred to as a probe 1.

3. The primer set for methylation of specific regions of human colorectal cancer-related genes according to claim 1, wherein
a detection primer set for the FOXE1 gene has a specific primer-probe set having the following sequences:
a forward primer: 5'- CGAGTttAAGtttTTGGtAGAGG -3', referred to as a primer 3;
a reverse primer: 5'- CCGATCGACTaAaaCCCGa -3', referred to as a primer 4; and
a detection probe: 5'- tCGAGTTCGGGCGtTGAGG -3', referred to as a probe 2.

4. The primer set for methylation of specific regions of human colorectal cancer-related genes according to claim 1, wherein
a detection primer set for the B3GAT2 gene has a specific primer-probe set having the following sequences:
a forward primer: 5'- TtCGtCGGGTtTGGCG -3', referred to as a primer 5;
a reverse primer: 5'- CAaaAaaTCGTaCAaCCCCG -3', referred to as a primer 6; and
a detection probe: 5'- tCGCGAGtAAGtTCGGGAG -3', referred to as a probe 3.

5. The primer set for methylation of specific regions of human colorectal cancer-related genes according to claim 1, wherein
a detection primer set for the Vimentin gene has a specific primer-probe set having the following sequences:
a forward primer: 5'- GtCGtAGttTtTACGttTCGT -3', referred to as a primer 7;
a reverse primer: 5'- aaCGCACGaCAaAaaAaCG -3', referred to as a primer 8; and
a detection probe: 5'- ttCGGGCGGCGTGTATG -3', referred to as a probe 4.

6. The primer set for methylation of specific regions of human colorectal cancer-related genes according to claim 1, wherein
a detection primer set for the SFRP2-1 gene has a specific primer-probe set having the following sequences:
a forward primer: 5'- CGGAtTGGGGtAAAAtAAGttC -3', referred to as a primer 9;
a reverse primer: 5'- CTaaCGaaAACGCGCCTA -3', referred to as a primer 10; and
a detection probe: 5'- TAGGCGCGTTttCGttAGTAttTGG -3', referred to as a probe 5.

7. The primer set for methylation of specific regions of human colorectal cancer-related genes according to claim 1, wherein
a detection primer set for the SFRP2-2 gene has a specific primer-probe set having the following sequences:
a forward primer: 5'- AATGtAGtCGGCGCG -3', referred to as a primer 11;
a reverse primer: 5'- CCTTaTTaaaAaTTCAAaaAaCCCG -3', referred to as a primer 12; and
a detection probe: 5'- AGttAtTTtCGGGCGTGCGGt -3', referred to as a probe 6.

8. The primer set for methylation of specific regions of human colorectal cancer-related genes according to claim 1, further comprising a primer set for an internal reference ACTB gene;
wherein
the primer set for the internal reference ACTB gene has a specific primer-probe set having the following sequences:
a forward primer: 5'- GTGATGGAGGAGGTTTAGTAAG -3', referred to as a primer 13;
a reverse primer: 5'- CAATAAAACCTACTCCTCCCTT -3', referred to as a primer 14; and
a detection probe: 5'- TGTGTTTGTTATTGTGTGTTGGGTGGT -3', referred to as a probe 7.

9. The primer set for methylation of specific regions of human colorectal cancer-related genes according to any one of claims 2-8, wherein
a fluorescent reporter group at a 5' end of the detection probe comprises: ALEX-350, FAM, VIC, TET, CAL Fluor Gold 540, JOE, HEX, CAL Flour Orange 560, TAMRA, Cal Fluor Red 590, ROX, CAL Fluor 20 Red 610, TEXAS RED, CAL Flour Red 635, Quasar 670, Cy3, Cy5, Cy5.5, or Quasar 705; and
a quenching group at 3' end of the detection probe comprises: TAMRA, BHQ1, BHQ2, and MGB.

10. A reagent for detecting methylation of specific regions of human colorectal cancer-related genes, comprising: six PCR systems, that are a CRC PCR Mix-A, a CRC PCR Mix-B, a CRC PCR Mix-C, a CRC PCR Mix-D, a CRC PCR Mix-E, and a CRC PCR Mix-F, respectively; wherein
each PCR system comprises:
the specific primer-probe set for the methylation of specific regions of a corresponding gene according to any one of claims 2-7;
the specific primer-probe set for the internal reference gene according to claim 8; and general ingredients, comprising: a PCR buffer, dNTPs, and MgCl₂.

11. The reagent for detecting methylation of specific regions of human colorectal cancer-related genes according to claim 10, wherein
in the six PCR systems of the CRC PCR Mix-A, the CRC PCR Mix-B, the CRC PCR Mix-C, the CRC PCR Mix-D, the CRC PCR Mix-E, and the CRC PCR Mix-F:
each reaction system has a final volume of between 20 and 40 µL;
primer concentrations of each reaction system are as follows:
the forward primer has a concentration of between 0.3 and 0.8 µM, the reverse primer has a concentration of between 0.3 and 0.8 µM, and the probe has a concentration of between 0.1 and 0.3 µM; and
final concentrations of the general ingredients of each of the six PCR systems are as follows:
final concentrations of dATP, dTTP, dCTP, and dGTP in the dNTPs are all between 0.1 and 0.3 mM, and a final concentration of MgCl₂ is between 1 and 2 mM.

12. A kit for detecting methylation of specific regions of human colorectal cancer-related genes, the kit comprising:
any one of the six PCR systems or any combination thereof according to claim 10;
between 0.5 and 2 U of a hot-start taq polymerase;
a positive control;
a negative control; and
a blank control;
wherein
the positive control is a mixture of the internal reference ACTB gene and methylated plasmid DNA fragments of PRDM12, FOXE1, B3GAT2, Vimentin, SFRP2-1, and SFRP2-2, or is a positive control of methylated human whole genome DNA;
the negative control is a mixture of the internal reference ACTB gene and unmethylated plasmid DNA fragments of PRDM12, FOXE1, B3GAT2, Vimentin, SFRP2-1, and SFRP2-2, or is a negative control of unmethylated human whole genome DNA; and
the blank control is a Tris-EDTA buffer or nuclease-free water having a pH = 8.5 ± 0.1.

13. A method of using the kit for detecting methylation of specific regions of human colorectal cancer-related genes according to claim 12, wherein
DNA is directly extracted from human feces, blood, or tissues, and the DNA is converted by bisulfite treatment, a resulting converted DNA is used as a template, amplified by PCR, and accumulation of signals on the detection probe is used to achieve real-time detection of methylation of specific regions of human colorectal cancer related genes of PRDM12, FOXE1, B3GAT2, Vimentin, SFRP2-1, and SFRP2-2 genes;
at the same time, the ACTB gene was used as the internal reference gene; and
the method comprises the following specific PCR experimental steps:
step 1: collecting feces, blood, or tissue samples from a certain number of colorectal cancer patients and advanced adenoma patients;
step 2: extracting human genomic DNA from the feces, blood or tissue samples of patients to be tested;
step 3: converting a resulting extracted human genomic DNA by bisulfite, and using a resulting converted DNA as the template for the PCR;
step 4: collecting 29.7 µL x(n+1) PCR Mix and 0.3µL ×(n+1) hot-start taq polymerase mixed solution from the detection kit, mixing uniformly for 20 s, and centrifuging instantaneously for 10 s; in which, n=number of samples to be tested;
step 5: dispensing a resulting centrifuged solution to PCR tubes with each PCR tube containing 30 µL of the resulting centrifuged solution, then adding 10 µL of the converted DNA template to be detected, wherein a total volume is 40 µL;
step 6: performing an amplification reaction, wherein the reaction procedure is as follows:
| stages | cycle number | reaction temperature | reaction time |
|---|---|---|---|
| first stage | 1 | 95.0°C | 1 min |
| second stage | 10 | 95.0°C | 20 s |
| | | 65.0°C | 30 s (-0.5°C/Cycler) |
| | | 72.0°C | 20 s |
| third stage | 35 | 95.0°C | 20 s |
| | | 60.0°C | 26 s (FAM, HEX, ROX, and Cy5 are lightened, and fluorescence value is detected once before the end of each cycle) |
| | | 72.0°C | 30 s |
step 5: determining PCR results, wherein during the sample detection, the baseline is determined by taking fluorescence signals of between 3 and 15 cycles; a threshold is set by making a threshold line exceeding a highest point of an amplification curve of a normal negative control, and the Ct value is Undet; positive or negative result of the sample is determined according to reported Ct values, that is, according to a cycle number experienced when the fluorescent signal in each reaction tube reaches the set threshold.
